# EUROPÄISCHE PATENTSCHRIFT

(11) **EP 1 755 562 B1**
(45) Veröffentlichungstag und Bekanntmachung des Hinweises auf die Patenterteilung: **09.10.2013**
(21) Anmeldenummer: 05750712.1
(22) Anmeldetag: 27.05.2005
(51) Int. Cl.: A61K 9/36, A61K 31/565

(54) **FOLSÄUREHALTIGES KONTRAZEPTIVUM**
CONTRACEPTIVE CONTAINING FOLIC ACID
CONTRACEPTIF CONTENANT DE L'ACIDE FOLIQUE

(30) Priorität: 28.05.2004 DE 102004026671; 28.05.2004 DE 102004026670
(43) Veröffentlichungstag der Anmeldung: 28.02.2007
(60) Teilanmeldung: 10008750.1
(73) Patentinhaber: Richter Gedeon Nyrt., 1103 Budapest (HU)
(72) Erfinder: SCHRAMM, Georg, 52224 Stolberg (DE); PÂQUES, Eric-Paul, 52076 Aachen (DE)
(74) Vertreter: Kutzenberger, Helga
(86) Internationale Anmeldenummer: PCT/EP2005/005761
(87) Internationale Veröffentlichungsnummer: WO 2005/115349

(56) Entgegenhaltungen:
- WO-A-99/53910
- GB-A- 888 631
- US-A1- 2003 050 287
- MOOIJ P N ET AL: "Multivitamin supplementation in oral contraceptive users." CONTRACEPTION. SEP 1991, Bd. 44, Nr. 3, September 1991 (1991-09), Seiten 277-288, XP002339644 ISSN: 0010-7824

## Beschreibung

Die vorliegende Erfindung betrifft eine Darreichungsform zur hormonalen Kontrazeption enthaltend eine bestimmte Anzahl von hormonhaltigen Tages-Einheiten und eine bestimmte Anzahl von hormonfreien Tages-Einheiten zur ununterbrochenen, täglichen, oralen Verabreichung an Frauen, dadurch gekennzeichnet, dass die hormonhaltigen Tages-Einheiten Folsäure in einer Tagesmenge von 0 bis 200 µg und die hormonfreien Tages-Einheiten jeweils Folsäure in einer Tagesmenge > 200 µg bis zu 5 mg an Folsäure enthalten.

Es wird vermutet, dass eine lang andauernde Einnahme von hormonalen Kontrazeptiva auf Basis von Gestagenen zu einem Mangel an Folsäure führen kann. Dieser Mangel kann beispielsweise zu cardiovaskulären Erkrankungen führen.

Außerdem ist bekannt, dass, sofern es kurze Zeit nach Abbruch der Einnahme solcher hormonaler Kontrazeptiva zu einer Schwangerschaft kommt, die Gefahr besteht, dass der Mangel an Folsäure bei dem Embryo zu Neuralrohrdefekten führen kann. Da das Neuralrohr in den ersten Wochen der Schwangerschaft entwickelt wird, ist es besonders vorteilhaft, eine präkonzeptionelle Einnahme von Folsäure zu gewährleisten.

Wenn daher wegen eines Kinderwunsches die Einnahme von hormonalen Kontrazeptiva unterbrochen wird und es bereits im ersten Zyklus nach dem Absetzen der hormonalen Kontrazeptiva zu einer Schwangerschaft kommt, ist die Gewährleistung einer entsprechend hohen Menge an Folsäure für die Zeit direkt nach dem Absetzen der sogenannten Pille besonders wichtig.

Es besteht daher ein Bedarf, hormonale Kontrazeptiva mit Folsäure in einer solchen Art und Weise auszurüsten, dass der Folsäure-Zusatz den unterschiedlichen Bedürfnissen über die Zeit während eines Einnahmezyklus und danach angepasst ist.

Aus WO 99/53910 ist bereits die Kombination von hormonalen Kontrazeptiva und Folsäure bekannt. Die Folsäuremenge pro hormonaler Tagesdosis wird nur entsprechend dem unterschiedlichen Bedarf an Folsäure gemäß fortschreitendem Alter geändert. Es wird aber nicht der unterschiedliche Bedarf an Folsäure über den Einnahmezyklus eines Kontrazeptivums berücksichtigt.

Daher war es Aufgabe der vorliegenden Erfindung, eine Darreichungsform zur hormonalen Kontrazeption zur Verfügung zu stellen, die den unterschiedlichen Bedarf an Folsäure während des hormonalen Einnahmezyklus und einer darauffolgenden Einnahme hormonfreier Tages-Einheiten berückslchtigt.

Diese Aufgabe wurde durch die Bereitstellung der erfindungsgemäßen Darreichungsform zur hormonalen Kontrazeption enthaltend eine bestimmte Anzahl von hormonhaltigen Tages-Einheiten und eine bestimmte Anzahl von hormonfreien Tages-Einheiten zur ununterbrochenen, täglichen, oralen Verabreichung an Frauen, dadurch gekennzeichnet, dass die hormonhaltigen Tages-Einheiten Folsäure in einer Tagesmenge von 0 bis 200 µg und die hormonfreien Tages-Einheiten jeweils Folsäure in einer Tagesmenge > 200 µg bis zu 5 mg an Folsäure enthalten, gelöst.

Frauen im gebärfähigen Alter haben einen täglichen Bedarf an Folsäure, der durch eine gesunde Ernährung ausreichend gedeckt werden kann. Eine lang andauernde Einnahme von hormonalen Kontrazeptiva enthaltend Gestagene kann zu einem zusätzlichen Bedarf an Folsäure führen, der ebenfalls durch eine gesunde Ernährung gedeckt werden kann. Allerdings ist dafür eine tägliche Gabe der bei Frauen minimal wirksamen Tagesmenge an Folsäure empfehlenswert.

Vorzugsweise enthalten die hormonhaltigen Tages-Einheiten der erfindungsgemäßen Darreichungsform 0 bis 200 µg Folsäure, besonders bevorzugt 5 bis 200 µg Folsäure.

Die hormonhaltigen Tages-Einheiten der erfindungsgemäßen Darreichungsform können auch keinen Zusatz an Folsäure aufweisen, wobei aber ein Zusatz von Folsäure bevorzugt ist.

Um bei einem angestrebten Kinderwunsch möglichst rasch die notwendige Menge an Folsäure bzw. den erhöhten Bedarf an Folsäure zumindest bei Beginn einer Schwangerschaft zu gewährleisten, und dabei einer mögliche Schädigung des Embryos durch Folsäuremangel vorzubeugen, enthalten die hormonfreien Tages-Einheiten der erfindungsgemäßen Darreichungsform Folsäure in einer Menge von mehr als 200 µg bis zur maximal zulässigen Tagesmenge an Folsäure für Frauen, vorzugsweise bis zu 5 mg Folsäure pro Tages-Einheit, besonders bevorzugt von mehr als 200 µg bis 5 mg Folsäure, ganz besonders bevorzugt bis zu der bei fertilen Frauen maximal zulässige Tagesmenge an Folsäure.

Durch den Zusatz von Folsäure zu den hormonfreien Tageseinheiten der erfindungsgemäßen Darreichungsform in Mengen bis zur maximal für fertile Frauen zulässigen Menge kann schon während der Einnahme der hormonfreien Tages-Einheiten die Folsäurekonzentration im weiblichen Körper soweit erhöht sein, dass gegebenenfalls bei einem Absetzen der Einnahme eines hormonhaltigen Kontrazeptivums und einer darauffolgenden Schwangerschaft möglichst frühzeitig der zu Beginn einer Schwangerschaft erhöhte Folsäurebedarf im weiblichen Körper gewährleistet ist.

Vorzugsweise enthalten die hormonhaltigen Tages-Einheiten der erfindungsgemäßen Darreichungsform jeweils dieselbe Menge Folsäure. Dies gilt auch für die hormonfreien Tages-Einheiten, die ebenfalls jeweils dieselbe, gegenüber den hormonhaltigen Tages-Einheiten aber erhöhte Menge Folsäure aufweisen.

Die Folsäure kann in der erfindungsgemäßen Darreichungsform auch als pharmazeutisch unbedenkliches Salz, vorzugsweise als Natrium-, Kalium- oder Magnesium-Salz oder als ein entsprechendes Derivat vorliegen.

Als Derivate der Folsäure eignen sich Mono- oder Diester, wobei bei Diestern eine unterschiedliche oder identische Veresterung vorliegen kann. Vorzugsweise eignet sich als Alkoholwert eine niedrige Alkylgruppe mit C₁-C₈, wie Methyl, Ethyl, Propyl oder Butyl, eine verzweigte, niedrige Alkylgruppe mit C₃-C₈, wie Isopropyl, Isobutyl oder sec. Butyl, eine Cycloalkyl-Gruppe, wie Cyclopentyl oder Cyclohexyl, eine Aryl-Gruppe, wie Phenyl oder substituiertes Phenyl mit 1-2 Substituenten, z. B. wie eine niedrige Alkyl- oder Halogen-Alkoxyl-Gruppe, oder eine Arylalkyl-Gruppe mit einem C₁-C₈ Alkylrest und eine Arylgruppe, wie Phenyl oder substituiertes Phenyl.

Darüber hinaus können die hormonfreien Tages-Einheiten und gegebenenfalls die hormonhaltigen Tages-Einheiten zusätzlich zur Folsäure weitere Vitamine oder Mineralien enthalten.

Die Anzahl der Tages-Einheiten einer erfindungsgemäßen Darreichungsform kann einem natürlichen, monatlichen, weiblichen Menstruations-Zyklus entsprechen. In diesem Fall enthält die erfindungsgemäße Darreichungsform 21 bis 25 hormonhaltige Tages-Einheiten und 7 bis 3 hormonfreie Tages-Einheiten.

Es ist aber auch möglich, dass die Gesamtzahl der hormonhaltigen Tages-Einheiten mehr als einen natürlichen, weiblichen Monatszyklus entspricht, so dass eine erfindungsgemäße Darreichungsform hormonhaltige Tages-Einheiten für eine ununterbrochene Einnahme bis zu 2 Jahren, vorzugsweise bis zu 1 Jahr, und 7 bis 3 hormonfreie Tages-Einheiten enthalten kann. Es ist aber auch möglich, dass die erfindungsgemäße Darreichungsform 42 bis 52 bzw. 77 bis 193 hormonhaltige Tages-Einheiten neben 7 bis 3 hormonfreien Tages-Einheiten aufweisen kann.

Die hormonhaltigen Tages-Einheiten der erfindungsgemäßen Darreichungsform können jeweils einen Gehalt von mindestens einer kontrazeptiv wirkenden Hormonkomponente, vorzugsweise eine Kombination von Hormonkomponenten wie einem Östrogen und einem Gestagen aufweisen.

Für die hormonhaltigen Tages-Einheiten der erfindungsgemäßen Darreichungsform eignen sich Östrogene, die vorzugsweise aus der Gruppe umfassend Oestradiol, Estradiolvalerat, Ethinylestradiol und Mestranol ausgewählt sind. Als Östrogen für die erfindungsgemäße Darreichungsform ist Ethinylestradiol besonders bevorzugt.

Für die hormonhaltigen Tages-Einheiten der erfindungsgemäßen Darreichungsform eignen sich Gestagene, die vorzugsweise aus der Gruppe umfassend Norethisteron, Norethisteronacetat, Norethisteronenantat, Norgestimat, Norgestrel, Levonorgestrel, Gestoden, Hydroxyprogesteroncaproat, Medroxyprogesteronacetat, Megestrolacetat, Chlormadinonacetat, Lynestrenol, Cyproteronacetat, Drospirenon, Dienogest, Desogestrel, Progesteron, Dydrogesteron, Medrogeston, Ethynodiol, Promegeston, Nomegestrolacetat und Trimegeston ausgewählt sind.

Die Hormone werden vorzugsweise in den nachstehend angegebenen Mengen verwendet.

### Östrogene:

| | |
|---|---|
| Estradiol, Estradiolvalerat | 0,5 bis 4 mg |
| Ethinylestradiol | 5 bis 50 µg |
| Mestranol | 8 bis 70 µg |

| Gestagene: | |
|---|---|
| Norethisteron, -acetat | 0,5 bis 1,0 mg |
| Norgestimat | 0,1 bis 0,25 mg |
| Norgestrel | 0,3 bis 1,0 mg |
| Levonorgestrel | 0,05 bis 0,15 mg |
| Gestoden | 0,05 bis 0,12 mg |
| Hydroxyprogesteroncaproat | 10 bis 800 mg |
| Medroxyprogesteronacetat | 2,5 bis 40 mg |
| Megestrolacetat | 1,0 bis 10 mg |
| Chlormadinonacetat | 0,5 bis 10 mg |
| Lynestrenol | 0,4 bis 3 mg |
| Cyproteronacetat | 0,5 bis 10 mg |
| Drospirenon | 1,0 bis 10 mg |
| Dienogest | 1,0 bis 10 mg |
| Desogestrel | 0,06 bis 0,30 mg |
| Progesteron | 100 bis 1000 mg |
| Dydrogesteron | 5 bis 50 mg |
| Medrogeston | 2 bis 30 mg |
| Ethynodiol, -diacetat | 0,4 bis 3 mg |
| Promegeston | 0,5 bis 10 mg |
| Nomegestrolacetat | 0,5 bis 10 mg |
| Trimegeston | 0,1 bis 10 mg |
| Etonogestrel | 0,1 bis 1 mg |
| Norelgestromin | 0,1 bis 2 mg |
| Norethynodrel | 0,3 bis 3 mg |
| Tibolon | 1 bis 10 mg |

Vorzugsweise weisen die erfindungsgemäßen Darreichungsformen, insbesondere die hormonhaltigen Tages-Einheiten, folgende Hormonkombinationen auf:

| | |
|---|---|
| 1. | 0,015 mg Ethinylestradiol + |
| | 0,06 mg Gestoden |
| 2. | 0,02 mg Ethinylestradiol + |
| | 0,15 mg Desogestrel |
| 3. | 0,02 mg Ethinylestradiol + |
| | 0,5 mg Norethisteron |
| 4. | 0,02 mg Ethinylestradiol + |
| | 1 mg Chlormadiononacetat oder 2 mg oder 3 mg Chlormadinonacetat |
| 5. | 0,02 mg Ethinylestradiol + |
| | 1 mg Norethisteron |
| 6. | 0,03 mg Ethinylestradiol + |
| | 1 mg Norethisteron |
| 7. | 0,02 mg Ethinylestradiol + |
| | 4 mg Chlormadinonacetat |
| 8. | 0,02 mg Ethinylestradiol + |
| | 5 mg Chlormadinonacetat |
| 9. | 0,02 mg Ethinylestradiol + |
| | 0,1 mg Levonorgestrel |
| 10. | 0,02 mg Ethinylestradiol + |
| | 0,15 mg Desogestrel |
| 11. | 0,02 mg Ethinylestradiol + |
| | 0,1 mg Levonorgestrel |
| 12. | 0,03 mg Ethinylestradiol + |
| | 3 mg Drospirenon |
| 13. | 0,02 mg Ethinylestradiol + |
| | 3 mg Drospirenon |
| 14. | 0,03 mg Ethinylestradiol + |
| | 2 mg Chlormadinonacetat |
| 15. | 0,035 mg Ethinylestradiol + |
| | 0,25 mg Norgestimat |
| 16. | 0,03 mg Ethinylestradiol + |
| | 0,5 mg Norethisteron |
| 17. | 0,03 mg Ethinylestradiol + |
| | 0,15 mg Desogestrel |
| 18. | 0,03 mg Ethinylestradiol + |
| | 0,075 mg Gestoden |
| 19. | 0,03 mg Ethinylestradiol + |
| | 0,15 mg Levonorgestrel |
| 20. | 0,03 mg Ethinylestradiol + |
| | 0,15 mg Desogestrel |
| 21. | 0,03 mg Ethinylestradiol + |
| | 0,15 mg Levonorgestrel |
| 22. | 0,03 mg Ethinylestradiol + |
| | 0,125 mg Levonorgestrel |
| 23. | 0,0375 mg Ethinylestradiol + |
| | 0, 75 mg Lynestrenol |
| 24. | 0,03 mg Ethinylestradiol + |
| | 1 mg Norethisteron |
| 25. | 0,03 mg Ethinylestradiol + |
| | 0,5 mg Norethisteron |
| 26. | 0,03 mg Ethinylestradiol + |
| | 0,15 mg Levonorgestrel |
| 27. | 0,04 mg Ethinylestradiol + |
| | 2 mg Lynestrenol |
| 28. | 1. Phase = 7 Tage |
| | 0,050 mg Desogestrel + 0,035 mg Ethinylestradiol |
| | 2. Phase = 7 Tage |
| | 0,100 mg Desogestrel + 0,030 mg Ethinylestradiol |
| | 3. Phase = 7 Tage |
| | 0,150 mg Desogestrel + 0,030 mg Ethinylestradiol29. |
| 29. | 1. Phase = 6 Tage |
| | 0,03 mg EE + 0,05 mg Levonorgestrel |
| | 2. Phase = 5 Tage |
| | 0,04 mg EE + 0,075 mg Levonorgestrel |
| | 3. Phase = 10 Tage |
| | 0,03 mg EE + 0,125 mg Levonorgestrel |
| 30. | 1. Phase = 7 Tage |
| | 0,035 mg EE + 0,180 mg Norgestimat |
| | 2. Phase = 7 Tage |
| | 0,035 mg EE + 0,215 mg Norgestimat |
| | 3. Phase = 7 Tage |
| | 0,035 mg EE + 0,250 mg Norgestimat |
| 31. | 1. Phase = 7 Tage |
| | 0,035 mg EE + 0,5 mg Norethisteron |
| | 2. Phase = 9 Tage |
| | 0,035 mg EE + 1 mg Norethisteron |
| | 3. Phase = 5 Tage |
| | 0,035 mg EE + 0,5 mg Norethisteron |
| 32. | 1. Phase = 7 Tage |
| | 0,035 mg EE + 0,5 mg Norethisteron |
| | 2. Phase = 7 Tage |
| | 0,035 mg EE + 0,75 mg Norethisteron |
| | 3. Phase = 7 Tage |
| | 0,035 mg EE + 1 mg Norethisteron |
| 33. | 1. Phase = 6 Tage |
| | 0,03 mg EE + 0,05 mg Levonorgestrel |
| | 2. Phase = 6 Tage |
| | 0,04 mg EE + 0,075 mg Levonorgestrel |
| | 3. Phase = 9 Tage |
| | 0,03 mg EE + 0,125 mg Levonorgestrel |
| 34. | 1. Phase = 6 Tage |
| | 0,03 mg EE + 0,05 mg Levonorgestrel |
| | 2. Phase = 5 Tage |
| | 0,05 mg EE + 0,05 mg Levonorgestrel |
| | 3. Phase = 10 Tage |
| | 0,04 mg EE + 0,125 mg Levonorgestrel |
| 35. | 0,035 mg Ethinylestradiol + |
| | 2 mg Cyproteronacetat |
| 36. | 0,05 mg Mestranol + |
| | 2 mg Chlormadinonacetat |
| 37. | 1. Phase = 11 Tage |
| | 0,05 mg Ethinylestradiol + |
| | 1 mg Chlormadinonacetat |
| | 2. Phase = 11 Tage |
| | 0,05 mg Ethinylestradiol + |
| | 2 mg Chlormadinonacetat |
| 38. | 0,08 mg Mestranol + |
| | 2 mg Chlormadinonacetat |
| 39. | 0,03 mg Ethinylestradiol + |
| | 2 mg Dienogest |
| 40. | 0,05 mg Ethinylestradiol + |
| | 0,5 mg Norgestrel |
| 41. | 0,05 mg Ethinylestradiol + |
| | 0,125 mg Levonorgestrel |
| 42. | 0,05 mg Ethinylestradiol + |
| | 0,25 mg Levonorgestrel |
| 43. | 0,05 mg Ethinylestradiol + |
| | 1 mg Norethisteronacetat |
| 44. | 1. Phase = 7 Tage |
| | 0,04 mg Ethinylestradiol + |
| | 0,025 mg Desogestrel |
| | 2. Phase = 15 Tage |
| | 0,03 mg Ethinylestradiol + |
| | 0,125 mg Desogestrel |
| 45. | 1. Phase = 11 Tage |
| | 0,05 mg Ethinylestradiol + |
| | 0,05 mg Levonorgestrel |
| | 2. Phase = 10 Tage |
| | 0,05 mg Ethinylestradiol + |
| | 0,125 mg Levonorgestrel |
| 46. | 1. Phase = 7 Tage |
| | 0,05 mg Ethinylestradiol |
| | 2. Phase = 15 Tage |
| | 0,05 mg Ethinylestradiol + |
| | 2,5 mg Lynestrenol |
| 47. | 1. Phase = 7 Tage |
| | 0,05 mg Ethinylestradiol |
| | 2. Phase = 15 Tage |
| | 0,05 mg Ethinylestradiol + |
| | 0,125 mg Desogestrel |
| 48. | 1. Phase = 6 Tage |
| | 0,05 mg Ethinylestradiol |
| | 2. Phase = 15 Tage |
| | 0,05 mg Ethinylestradiol + |
| | 1 mg Norethisteronacetat |

Es wird empfohlen, die erfindungsgemäße Darreichungsform, sofern sie eine mehrphasige Hormonkombination vorsieht, nur für eine ununterbrochene Einnahme der hormonhaltigen Tages-Einheiten für die Dauer von 21 bis 25 Tagen, gefolgt von einer 7- bis 3-tägigen Einnahme von hormonfreien Tages-Einheiten vorzusehen.

Vorzugsweise weisen die hormonhaltigen Tages-Einheiten 5 bis 30 µg Ethinylestradiol und 0,5 bis 5 mg Chlormadinonacetat auf, wobei mindestens 21 Tages-Einheiten, vorzugsweise 21 bis 25 Tages-Einheiten und 3 bis 7 hormonfreie Tages-Einheiten, in der erfindungsgemäßen Darreichungsform vorliegen. Die erfindungsgemäße Darreichungsform kann aber auch hormonhaltige Tages-Einheiten für mehrere Jahre, vorzugsweise von 42 bis 365 Einheiten umfassen, die die genannte Hormonkombination in den angegebenen Mengenbereichen enthalten, wobei sich an die entsprechenden ununterbrochenen Einnahmezeiten 7 bis 3 hormonfreie Tages-Einheiten mit einer erfindungsgemäß angegebenen, erhöhten Menge Folsäure anschließen.

Wie bereits ausgeführt, können die hormonhaltigen Tages-Einheiten und die hormonfreien Tages-Einheiten der erfindungsgemäßen Darreichungsform als ein-(mono) oder mehrphasiges Kontrazeptivum aufgebaut sein. Bei einem mehrphasigen Kontrazeptivum kann eine Zweiphasen- oder eine Dreiphasen-Pille vorliegen, die sich aber nicht üblicherweise für eine über den natürlichen, weiblichen Zyklus hinausgehende Einnahmedauer eignet.

Die erfindungsgemäße Darreichungsform umfasst die Tages-Einheiten vorzugsweise in Form von Tabletten, die im Blister verpackt sind, die auch vorzugsweise eine Einnahme-Markierung aufweisen. Dies ist insbesondere von Vorteil, wenn die erfindungsgemäße Darreichungsform als ein Kontrazeptivum entsprechend dem weiblichen Menstruationszyklus vorgesehen ist.

Die erfindungsgemäße Darreichungsform kann auch ein Bestandteil eines Kits sein, wobei das erfindungsgemäße Kit mehrere der erfindungsgemäßen Darreichungsformen umfassen kann, insbesondere wenn eine Darreichungsform nur einen monatlichen Zyklus umfasst. Gegebenenfalls wird von dem Kit auch noch ein Kalender bzw. ein Kalenderbuch mit umfasst.

### Beispiele

### Beispiel 1:

### Zusammensetzung

| | a) | b) |
|---|---|---|
| | Pro Tablette | Pro Tablette |
| Ethinylestradiol | 0,020 mg | |
| Chlormadinonacetat | 2,000 mg | |
| Natriumfolat | 0,050 mg | 3,000 mg |
| Povidon K30 | 3,000 mg | 3,000 mg |
| Lactose | 31,930 mg | 31,000 mg |
| Maisstärke | 12,000 mg | 12,000 mg |
| Magnesiumstearat | 0,500 mg | 0,500 mg |
| hochdisperses Siliciumdioxid | 0,500 mg | 0,500 mg |

a) Ethinylestradiol (EE), Povidon K 30 (Polyvinylpyrrolidon) und das Natriumsalz der Folsäure wurden in 600 ml Ethanol gelöst. Chlormadinonacetat (Partikelgröße 90% < 50 µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 50 mg gepresst.
b) Wie unter a) angegeben, wurden hormonfreie, folsäurehaltige Tabletten mit einem Gewicht von 50 mg hergestellt, wobei das Natriumsalz der Folsäure in 600 ml wässrigem Ethanol gelöst wurde.

Die Tabletten a) bzw. b) wurden mit einem Lack auf Basis Methylhydroxypropylcellulose überzogen (z. B. Opadry YS-1-2184 vom Hersteller Colorcon); Überzugsmasse 2 mg pro Tablette.

Zur Herstellung eines erfindungsgemäßen Kontrazeptivums wurden 21 hormonhaltige Tabletten und 7 hormonfreie Tabletten als 28 Tages-Einheiten zu einem Blister verpackt.

## Patentansprüche

1. Darreichungsform zur hormonalen Kontrazeption enthaltend eine bestimmte Anzahl von hormonhaltigen Tages-Einheiten und eine bestimmte Anzahl von hormonfreien Tages-Einheiten zur ununterbrochenen, täglichen, oralen Verabreichung an Frauen, **dadurch gekennzeichnet, dass** die hormonhaltigen Tages-Einheiten Folsäure in einer Tagesmenge von jeweils 0 bis 200 µg und die hormonfreien Tages-Einheiten jeweils Folsäure in einer Tagesmenge von mehr als 200 µg bis zu 5 mg enthalten.

2. Darreichungsform nach Anspruch 1, **dadurch gekennzeichnet, dass** die harmonhaltigen Tages-Einheiten keine Folsäure enthalten.

3. Darreichungsform nach Anspruch 1, **dadurch gekennzeichnet, dass** die hormonhaltigen Tages-Einheiten jeweils 5 bis 200 µg Folsäure enthalten.

4. Darreichungsform nach einem der Ansprüche 1 bis 3, **dadurch gekennzeichnet, dass** die hormonhaltigen Tages-Einheiten jeweils dieselbe Menge Folsäure und die hormonfreien Tages-Einheiten ebenfalls jeweils dieselbe Menge Folsäure enthalten.

5. Darreichungsform nach einem der Ansprüche 1 bis 4, **dadurch gekennzeichnet, dass** sie wenigstens 21 hormonhaltige Tages-Einheiten und 7 bis 3 hormonfreie Tages-Einheiten aufweist.

6. Darreichungsform nach Anspruch 5, **dadurch gekennzeichnet, dass** deren maximale Anzahl an hormonhaltigen Tages-Einheiten einer ununterbrochenen Verabreichung für mehrere Jahre, vorzugsweise für 2 Jahre, besonders bevorzugt für 1 Jahr, und deren Anzahl an hormonfreien Tages-Einheiten einer Verabreichung für 7 bis 3 Tage entspricht.

7. Darreichungsform nach Anspruch 5, **dadurch gekennzeichnet, dass** sie bis zu 730, vorzugsweise bis zu 365 hormonhaltige Tages-Einheiten zur ununterbrochenen Verabreichung und 7 bis 3 hormonfreie Tages-Einheiten aufweist.

8. Darreichungsform nach Anspruch 5, **dadurch gekennzeichnet, dass** sie 77 bis 193 hormonhaltige Tages-Einheiten zur ununterbrochenen Verabreichung und 7 bis 3 hormonfreie Tages-Einheiten aufweist.

9. Darreichungsform nach Anspruch 5, **dadurch gekennzeichnet, dass** sie 42 bis 52 hormonhaltige Tages-Einheiten zur ununterbrochenen Verabreichung und 7 bis 3 hormonfreie Tages-Einheiten aufweist.

10. Darreichungsform nach Anspruch 5, **dadurch gekennzeichnet, dass** sie 21 bis 25 hormonhaltige Tages-Einheiten und 7 bis 3 hormonfreie Tages-Einheiten aufweist.

11. Darreichungsform nach einem der Ansprüche 1 bis 10, **dadurch gekennzeichnet, dass** die hormonhaltigen Tages-Einheiten jeweils einen Gehalt an mindestens einer kontrazeptiv wirkenden Hormonkomponente, vorzugsweise einer Kombination von Hormonkomponenten, besonders bevorzugt eine Kombination aus einem Östrogen und einem Gestagen, aufweisen.

12. Darreichungsform nach einem der Ansprüche 1 bis 11, **dadurch gekennzeichnet, dass** die Anzahl der hormonhaltigen Tages-Einheiten einem monophasigen Kontrazeptivum entspricht.

13. Darreichungsform nach Anspruch 11, **dadurch gekennzeichnet, dass** eine hormonhaltige Tages-Einheit ein Östrogen aus der Gruppe umfassend Oestradiol, Estradiolvalerat, Ethinylestradiol, Mestranol und ein Gestagen aus der Gruppe umfassend Norethisteron, Norethisteronacetat, Norethisteronenantat, Norgestimat, Norgestrel, Levonorgestrel, Gestoden, Hydroxyprogesteroncaproat, Medroxyprogesteronacetat, Megestrolacetat, Chlormadinonacetat, Lynestrenol, Cyproteronacetat, Drospirenon, Dienogest, Desogestrel, Progesteron, Dydrogesteron, Medrogeston, Ethynodiol, Promegeston, Nomegestrolacetat und Trimegeston enthält.

14. Darreichungsform nach Anspruch 12, **dadurch gekennzeichnet**, das eine hormonhaltige Tages-Einheit eine Hormonkombination ausgewählt aus der Gruppe umfassend
| | |
|---|---|
| **1.** | 0,015 mg Ethinylestradiol + |
| | 0,06 mg Gestoden |
| **2.** | 0,02 mg Ethinylestradiol + |
| | 0,15 mg Desogestrel |
| **3.** | 0,02 mg Ethinylestradiol + |
| | 0,5 mg Norethisteron |
| **4.** | 0,02 mg Ethinylestradiol + |
| | 1 mg Chlormadiononacetat oder 2 mg oder 3 mg Chlormadinonacetat |
| **5.** | 0,02 mg Ethinylestradiol + |
| | 1 mg Norethisteron |
| **6.** | 0,03 mg Ethinylestradiol + |
| | 1 mg Norethisteron |
| **7.** | 0,02 mg Ethinylestradiol + |
| | 4 mg Chlormadinonacetat |
| **8.** | 0,02 mg Ethinylestradiol + |
| | 5 mg Chlormadinonacetat |
| **9.** | 0,02 mg Ethinylestradiol + |
| | 0,1 mg Levonorgestrel |
| **10.** | 0,02 mg Ethinylestradiol + |
| | 0,15 mg Desogestrel |
| **11.** | 0,02 mg Ethinylestradiol + |
| | 0,1 mg Levonorgestrel |
| **12.** | 0,03 mg Ethinylestradiol + |
| | 3 mg Drospirenon |
| **13.** | 0,02 mg Ethinylestradiol + |
| | 3 mg Drospirenon |
| **14.** | 0,03 mg Ethinylestradiol + |
| | 2 mg Chlormadinonacetat |
| **15.** | 0,035 mg Ethinylestradiol + |
| | 0,25 mg Norgestimat |
| **16.** | 0,03 mg Ethinylestradiol + |
| | 0,5 mg Norethisteron |
| **17.** | 0,03 mg Ethinylestradiol + |
| | 0,15 mg Desogestrel |
| **18.** | 0,03 mg Ethinylestradiol + |
| | 0,075 mg Gestoden |
| **19.** | 0,03 mg Ethinylestradiol + |
| | 0,15 mg Levonorgestrel |
| **20.** | 0,03 mg Ethinylestradiol + |
| | 0,15 mg Desogestrel |
| **21.** | 0,03 mg Ethinylestradiol + |
| | 0,15 mg Levonorgestrel |
| **22.** | 0,03 mg Ethinylestradiol + |
| | 0,125 mg Levonorgestrel |
| **23.** | 0,0375 mg Ethinylestradiol + |
| | 0,75 mg Lynestrenol |
| **24.** | 0,03 mg Ethinylestradiol + |
| | 1 mg Norethisteron |
| **25.** | 0,03 mg Ethinylestradiol + |
| | 0,5 mg Norethisteron |
| **26.** | 0,03 mg Ethinylestradiol + |
| | 0,15 mg Levonorgestrel oder |
| **27.** | 0,04 mg Ethinylestradiol + 2 mg Lynestrenol |

15. Ein Kit enthaltend mindestens eine Darreichungsform zur hormonalen Kontrazeption nach einem der Ansprüche 1 bis 14.

16. Ein Kit nach Anspruch 15, **dadurch gekennzeichnet, dass** es mehrere Darreichungsformen nach einem der Ansprüche 1 bis 14 enthält.

## Claims

1. Dosage form for hormonal contraception containing a specific number of hormone-containing daily units and a specific number of hormone-free daily units for uninterrupted daily oral administration to women, **characterised in that** the hormone-containing daily units contain folic acid in a respective daily amount of 0 to 200 µg and the hormone-free daily units respectively contain folic acid in a daily amount ranging from more than 200 µg up to 5 mg.

2. Dosage form according to claim 1, **characterised in that** the hormone-containing daily units do not contain any folic acid.

3. Dosage form according to claim 1, **characterised in that** the hormone-containing daily units respectively contain 5 to 200 µg folic acid.

4. Dosage form according to one of claims 1 to 3, **characterised in that** the hormone-containing daily units each contain the same amount of folic acid and the hormone-free daily units likewise each contain the same amount of folic acid.

5. Dosage form according to one of claims 1 to 4, **characterised in that** it comprises at least 21 hormone-containing daily units and 7 to 3 hormone-free daily units.

6. Dosage form according to claim 5, **characterised in that** its maximum number of hormone-containing daily units corresponds to uninterrupted administration for several years, preferably for 2 years, particularly preferred for 1 year, and the number of hormone-free daily units corresponds to administration for 7 to 3 days.

7. Dosage form according to claim 5, **characterised in that** it comprises up to 730, preferably up to 365, hormone-containing daily units for uninterrupted administration and 7 to 3 hormone-free daily units.

8. Dosage form according to claim 5, **characterised in that** it comprises 77 to 193 hormone-containing daily units for uninterrupted administration and 7 to 3 hormone-free daily units.

9. Dosage form according to claim 5, **characterised in that** it comprises 42 to 52 hormone-containing daily units for uninterrupted administration and 7 to 3 hormone-free daily units.

10. Dosage form according to claim 5, **characterised in that** it comprises 21 to 25 hormone-containing daily units and 7 to 3 hormone-free daily units.

11. Dosage form according to one of claims 1 to 10, **characterised in that** the hormone-containing daily units each contain at least one contraceptively active hormone component, preferably a combination of hormone components, particularly preferred a combination of an oestrogen and a progestogen.

12. Dosage form according to one of claims 1 to 11, **characterised in that** the number of hormone-containing daily units corresponds to a monophasic contraceptive.

13. Dosage form according to claim 11, **characterised in that** a hormone-containing daily unit contains an oestrogen from the group comprising oestradiol, oestradiol valerate ethinyloestradiol, mestranol and a progestogen from the group comprising norethisterone, norethisterone acetate, norethisterone enanthate, norgestimate, norgestrel, levonorgestrel, gestodene, hydroxyprogesterone caproate, medroxyprogesterone acetate, megestrol acetate, chlormadinone acetate, lynestrenol, cyproterone acetate, drospirenone, dienogest, desogestrel, progesterone, dydrogesterone, medrogestone, ethynodiol, promegestone, nomegestrol acetate and trimegestone.

14. Dosage form according to claim 12, **characterised in that** a hormone-containing daily unit contains a hormone combination selected from the group comprising
| | |
|---|---|
| 1. | 0.015 mg ethinyloestradiol + |
| | 0.06 mg gestoden |
| 2. | 0.02 mg ethinyloestradiol + |
| | 0.15 mg desogestrel |
| 3. | 0.02 mg ethinyloestradiol + |
| | 0.5 mg norethisterone |
| 4. | 0.02 mg ethinyloestradiol + |
| | 1 mg chlormadinone acetate or 2 mg or 3 mg chlormadinone acetate |
| 5. | 0.02 mg ethinyloestradiol + |
| | 1 mg norethisterone |
| 6. | 0.03 mg ethinyloestradiol + |
| | 1 mg norethisterone |
| 7. | 0.02 mg ethinyloestradiol + |
| | 4 mg chlormadinone acetate |
| 8. | 0.02 mg ethinyloestradiol + |
| | 5 mg chlormadinone acetate |
| 9. | 0.02 mg ethinyloestradiol + |
| | 0.1 mg levonorgestrel |
| 10. | 0.02 mg ethinyloestradiol + |
| | 0.15 mg desogestrel |
| 11. | 0.02 mg ethinyloestradiol + |
| | 0.1 mg levonorgestrel |
| 12. | 0.03 mg ethinyloestradiol + |
| | 3 mg drospirenone |
| 13. | 0.02 mg ethinyloestradiol + |
| | 3 mg drospirenone |
| 14. | 0.03 mg ethinyloestradiol + |
| | 2 mg chlormadinone acetate |
| 15. | 0.035 mg ethinyloestradiol + |
| | 0.25 mg norgestimate |
| 16. | 0.03 mg ethinyloestradiol + |
| | 0.5 mg norethisterone |
| 17. | 0.03 mg ethinyloestradiol + |
| | 0.15 mg desogestrel |
| 18. | 0.03 mg ethinyloestradiol + |
| | 0.075 mg gestodene |
| 19. | 0.03 mg ethinyloestradiol + |
| | 0.15 mg levonorgestrel |
| 20. | 0.03 mg ethinyloestradiol + |
| | 0.15 mg desogestrel |
| 21. | 0.03 mg ethinyloestradiol + |
| | 0.15 mg levonorgestrel |
| 22. | 0.03 mg ethinyloestradiol + |
| | 0.125 mg levonorgestrel |
| 23. | 0.0375 mg ethinyloestradiol + |
| | 0.75 mg lynestrenol |
| 24. | 0.03 mg ethinyloestradiol + |
| | 1 mg norethisterone |
| 25. | 0.03 mg ethinyloestradiol + |
| | 0.5 mg norethisterone |
| 26. | 0.03 mg ethinyloestradiol + |
| | 0.15 mg levonorgestrel |
| | or |
| 27. | 0.04 mg ethinyloestradiol + |
| | 2 mg lynestrenol. |

15. A kit containing at least one dosage form for hormonal contraception according to one of claims 1 to 14.

16. A kit according to claim 15, **characterised in that** it contains a plurality of dosage forms according to one of claims 1 to 14.

## Revendications

1. Forme galénique pour la contraception hormonale par voie orale, contenant un nombre déterminé d'unités journalières contenant des hormones et un nombre déterminé d'unités journalières sans hormones, pour l'administration ininterrompue, journalière par voie orale à des femmes, **caractérisée en ce que** les unités journalières contenant des hormones contiennent de l'acide folique en une quantité journalière à chaque fois de 0 à 200 µg et les unités journalières sans hormones contiennent à chaque fois de l'acide folique en une quantité journalière de plus de 200 µg jusqu'à 5 mg.

2. Forme galénique selon la revendication 1, **caractérisée en ce que** les unités journalières contenant des hormones ne contiennent pas d'acide folique.

3. Forme galénique selon la revendication 1, **caractérisée en ce que** les unités journalières contenant des hormones contiennent à chaque fois 5 à 200 µg d'acide folique.

4. Forme galénique selon l'une quelconque des revendications 1 à 3, **caractérisée en ce que** les unités journalières contenant des hormones contiennent à chaque fois la même quantité d'acide folique et les unités journalières sans hormones contiennent également à chaque fois la même quantité d'acide folique.

5. Forme galénique selon l'une quelconque des revendications 1 à 4, **caractérisée en ce qu'**elle présente au moins 21 unités journalières contenant des hormones et 7 à 3 unités journalières sans hormones.

6. Forme galénique selon la revendication 5, **caractérisée en ce que** son nombre maximal d'unités journalières contenant des hormones correspond à une administration ininterrompue pendant plusieurs années, de préférence pendant 2 années, de manière particulièrement préférée pendant 1 année et son nombre d'unités journalières sans hormones correspond à une administration pendant 7 à 3 jours.

7. Forme galénique selon la revendication 5, **caractérisée en ce qu'**elle présente jusqu'à 730, de préférence jusqu'à 365 unités journalières contenant des hormones pour l'administration ininterrompue et 7 à 3 unités journalières sans hormones.

8. Forme galénique selon la revendication 5, **caractérisée en ce qu'**elle présente 77 à 193 unités journalières contenant des hormones pour l'administration ininterrompue et 7 à 3 unités journalières sans hormones.

9. Forme galénique selon la revendication 5, **caractérisée en ce qu'**elle présente 42 à 52 unités journalières contenant des hormones pour l'administration ininterrompue et 7 à 3 unités journalières sans hormones.

10. Forme galénique selon la revendication 5, **caractérisée en ce qu'**elle présente 21 à 25 unités journalières contenant des hormones et 7 à 3 unités journalières sans hormones.

11. Forme galénique selon l'une quelconque des revendications 1 à 10, **caractérisée en ce que** les unités journalières contenant des hormones présentent à chaque fois une teneur en au moins un composant hormonal à effet contraceptif, de préférence une combinaison de composants hormonaux, de manière particulièrement préférée une combinaison d'un estrogène et d'un gestagène.

12. Forme galénique selon l'une quelconque des revendications 1 à 11, **caractérisée en ce que** le nombre d'unités journalières contenant des hormones correspond à un contraceptif monophasique.

13. Forme galénique selon la revendication 11, **caractérisée en ce qu'**une unité journalière contenant des hormones contient un estrogène du groupe comprenant l'estradiol, le valérate d'estradiol, l'éthinylestradiol, le mestranol et un gestagène du groupe comprenant la noréthistérone, l'acétate de noréthistérone, l'énanthate de noréthistérone, le norgestimate, le norgestrel, le lévonorgestrel, le gestodène, le caproate d'hydroxyprogestérone, l'acétate de médroxyprogestérone, l'acétate de mégestrol, l'acétate de chlormadinone, le lynestrénol, l'acétate de cyprotérone, la drospirénone, le diénogest, le désogestrel, la progestérone, la dydrogestérone, la médrogestone, l'éthynodiol, la promégestone, l'acétate de nomégestrol et la trimégestone.

14. Forme galénique selon la revendication 12, **caractérisée en ce qu'**une unité journalière contenant des hormones contient une combinaison hormonale choisie dans le groupe comprenant
| | |
|---|---|
| 1. | 0,015 mg d'éthinylestradiol + |
| | 0,06 mg de gestodène |
| 2. | 0,02 mg d'éthinylestradiol + |
| | 0,15 mg de désogestrel |
| 3. | 0,02 mg d'éthinylestradiol + |
| | 0,5 mg de noréthistérone |
| 4. | 0,02 mg d'éthinylestradiol + |
| | 1 mg d'acétate de chlormadinone ou 2 mg ou 3 mg d'acétate de chlormadinone |
| 5. | 0,02 mg d'éthinylestradiol + |
| | 1 mg de noréthistérone |
| 6. | 0,03 mg d'éthinylestradiol + |
| | 1 mg de noréthistérone |
| 7. | 0,02 mg d'éthinylestradiol + |
| | 4 mg d'acétate de chlormadinone |
| 8. | 0,02 mg d'éthinylestradiol + |
| | 5 mg d'acétate de chlormadinone |
| 9. | 0,02 mg d'éthinylestradiol + |
| | 0,1 mg de lévonorgestrel |
| 10. | 0,02 mg d'éthinylestradiol + |
| | 0,15 mg de désogestrel |
| 11. | 0,02 mg d'éthinylestradiol + |
| | 0,1 mg de lévonorgestrel |
| 12. | 0,03 mg d'éthinylestradiol + |
| | 3 mg de drospirénone |
| 13. | 0,02 mg d'éthinylestradiol + |
| | 3 mg de drospirénone |
| 14. | 0,03 mg d'éthinylestradiol + |
| | 2 mg d'acétate de chlormadinone |
| 15. | 0,035 mg d'éthinylestradiol + |
| | 0,25 mg de norgestimate |
| 16. | 0,03 mg d'éthinylestradiol + |
| | 0,5 mg de noréthistérone |
| 17. | 0,03 mg d'éthinylestradiol + |
| | 0,15 mg de désogestrel |
| 18. | 0,03 mg d'éthinylestradiol + |
| | 0,075 mg de gestodène |
| 19. | 0,03 mg d'éthinylestradiol + |
| | 0,15 mg de lévonorgestrel |
| 20. | 0,03 mg d'éthinylestradiol + |
| | 0,15 mg de désogestrel |
| 21. | 0,03 mg d'éthinylestradiol + |
| | 0,15 mg de lévonorgestrel |
| 22. | 0,03 mg d'éthinylestradiol + |
| | 0,125 mg de lévonorgestrel |
| 23. | 0,0375 mg d'éthinylestradiol + |
| | 0,75 mg de lynestrénol |
| 24. | 0,03 mg d'éthinylestradiol + |
| | 1 mg de noréthistérone |
| 25. | 0,03 mg d'éthinylestradiol + |
| | 0,5 mg de noréthistérone |
| 26. | 0,03 mg d'éthinylestradiol + |
| | 0,15 mg de lévonorgestrel ou |
| 27. | 0,04 mg d'éthinylestradiol + |
| | 2 mg de lynestrénol |

15. Kit contenant au moins une forme galénique pour la contraception hormonale selon l'une quelconque des revendications 1 à 14.

16. Kit selon la revendication 15, **caractérisé en ce qu'**il contient plusieurs formes galéniques selon l'une quelconque des revendications 1 à 14.
